# EUROPEAN PATENT SPECIFICATION

(11) **EP 2 015 826 B1**
(45) Date of publication and mention of the grant of the patent: **02.11.2011**
(21) Application number: 07776058.5
(22) Date of filing: 23.04.2007
(51) Int. Cl.: A61M 25/09, A61M 25/06, A61M 25/01

(54) **GUIDEWIRE PLACEMENT DEVICE**
VORRICHTUNG ZUR FÜHRUNGSDRAHTPOSITIONIERUNG
DISPOSITIF DE PLACEMENT DE FILS-GUIDES

(30) Priority: 21.04.2006 US 793787 P
(43) Date of publication of application: 21.01.2009
(73) Proprietor: Abbott Laboratories, Abbott Park, Illinois 60064-3502 (US)
(72) Inventor: VON OEPEN, Randolf, Los Altos, California 94024 (US); COFFEY, Lorcan, 72076 Tubingen (DE); NEWHAUSER, Richard, Redwood City, California 94062 (US); RIETH, Thomas, 72145 Hirrlingen (DE); YRIBARREN, Travis, San Mateo, California 94401 (US)
(74) Representative: ZBM Patents
(86) International application number: PCT/US2007/009888
(87) International publication number: WO 2007/124161

(56) References cited:
- WO-A-2004/064891
- US-A- 4 601 713
- US-A1- 2002 133 217
- US-A1- 2003 120 208
- US-A1- 2004 068 250

## Description

### BACKGROUND OF THE INVENTION

### Field of the Invention

This invention relates to devices for facilitating the delivery of multiple guidewires within a vascular system. More particularly, the present invention is directed to a device having an elongated body with a plurality of guidewire channels configured for placing guidewires in the branches of a vascular system using a device in accordance with the present invention, while mitigating guidewire entanglement.

### Description of Related Art

Percutaneous transluminal coronary angioplasty (PTCA) is a procedure that is well known for the treatment of blockages in the coronary arteries. Blockages may occur from cholesterol precipitation on the coronary wall that may be in any stage from initial deposit through aged lesions. Coronary arteries may also become blocked due to the formation of thrombus. A widely used form of percutaneous coronary angioplasty makes use of a dilatation balloon catheter that is introduced into and advanced through a lumen or body vessel until the distal end thereof is at a desired location in the vessel system. Once in position across a lesion site, the expandable portion of the catheter, or balloon, is inflated to a predetermined size with a fluid at relatively high pressures, to radially compress the atherosclerotic plaque of the lesion against the inside of the artery wall and thereby dilate the lumen of the artery. The balloon is then deflated to a small profile so that the dilatation catheter may be withdrawn from the patient's vessel system and blood flow resumed through the dilated artery.

In angioplasty procedures of the kind described above, there may be restenosis of the artery, which either necessitates a subsequent angioplasty procedure, surgical by-pass operation, or some method of repairing or strengthening the area. To reduce restenosis and strengthen the area, a physician can implant an intravascular prosthesis for maintaining vascular patency, such as a stent, inside the artery at the lesion.

Stents, grafts, stent-grafts, filters, vena cava filters and similar implantable medical devices, collectively referred to hereinafter as stents, are radially expandable endoprostheses which are typically intravascular implants capable of being implanted transluminally and enlarged radially after being introduced percutaneously. Stents may be implanted in a variety of body lumens or vessels such as within the vascular system, urinary tracts, bile ducts, etc. Stents may be used to reinforce body vessels and to prevent restenosis following angioplasty in the vascular system. They may be self-expanding, such as nitinol shape memory stents, mechanically expandable, such as balloon expandable stents, expanded from a collapsed stage to an open stage by means of a balloon or other mechanism and maintain the open stage by being cured or hardened, or hybrid expandable.

US 2002/0133217 describes an intravascular balloon catheter which comprises a tubular catheter body having a balloon structure removably mounted over the catheter body. The catheter body has a guidewire lumen, and the catheter body may be left in place within a patient's vasculature.

US 2004/0068250 discloses a rapid exchange catheter comprising a guide wire lumen including a substantially sealed portion in which a lumen wall extends around an entire periphery thereof and a channel portion including a channel opening the lumen to an exterior of the catheter, wherein a width of the channel is less than a maximum width of the channel portion.

Although PTCA is widely known and used, some PTCA cases remain more challenging than others. For instance, the complexity of the case is increased when the desired location for placement of the stent is also the site of a bifurcation in the vessel system. In these cases, access to both vessel branches is preferably maintained by placing a guidewire in each branch. Typically, each guidewire is placed prior to advancement of the stent or angioplasty device. Since the guidewires may have encountered tortuosity during delivery to their respective body vessels, it is possible that entanglements may have occurred between them. These entanglements can present difficulties for subsequent advancement of a stent or angioplasty device to the treatment site. For example, one guidewire may be twisted over the other multiple times, thereby effectively forming a knot in the guidewires.

In practice, there are techniques that can help prevent or correct the problems that arise from guidewire entanglement. For example, during advancement of the second guidewire into the appropriate vessel, the operator may take care to not introduce more than a 180-degree twist in the wire. This technique may prevent guidewire entanglement from occurring. Additionally, once the device is advanced, if guidewire entanglement prevents guidewire motion, one of the guidewires may be retracted to relieve the entanglements. The guidewire may then be re-advanced to the appropriate vessel location and the device can then be fully advanced to the desired location. These techniques may be used separately, in combination with each other, or in combination with other operator techniques to mitigate the problem of guidewire entanglement. However, this requires additional attention by the operator, and it complicates the interventional procedure. Therefore, there is still a need for a device and method to assist in guidewire placement and mitigate guidewire entanglement during medical interventional precesures.

This need is met by the present invention, which is directed toward devices and methods of using the devices to enable placement of one or more guidewires into the branches of a bifurcation while mitigating the problem of guidewire entanglement and knot formation.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### SUMMARY OF THE INVENTION

The purpose and advantages of the present invention will be described and apparent from the description that follows, and through the practice of the invention.

To achieve these purposes and advantages, and in accordance with the present invention, there exists a guidewire placement device that includes an elongated body having a distal end and proximal end, and two guidewire channels. The elongated body has an inner wall thickness to isolate each guidewire channel from another.

In further accordance with the present invention, the guidewire channels are defined by the inner wall thickness of the elongated body and an outer wall thickness of the elongated body. A discontinuity is formed in the outer wall thickness adjacent each guidewire channel to allow a guidewire to be removably placed in a guidewire channel. The discontinuity has a proximal terminus coincident with the proximal end of the elongated body and a distal terminus along the elongated body. A radiopaque marker is associated with the elongated body thereby permitting visualization of the catheter location within the vessel system.

In accordance with a further aspect of the invention, each discontinuity can span the entire length of the elongated body.

In accordance with an example, a method can be provided for forming a discontinuity in the outer wall thickness of the elongated body that includes the step of passing a sharpened blade through the outer wall thickness from one terminus to the other.

In accordance with a further example, a method can be provided for forming a discontinuity in the outer wall thickness of the elongated body that includes supporting the guidewire channel using a mandrel, passing the guidewire channel underneath a laser beam, water jet, or another focused form of energy that will suitably form a discontinuity in the outer wall thickness.

In accordance with a further aspect of the present invention, the distal end of the guidewire placement device may include an atraumatic tip to facilitate safe and effective tracking of the device through a tortuous anatomy.

In accordance with another aspect of the present invention, the radiopaque marker can be positioned adjacent to the distal tip of the guidewire placement device to function as a marker during the PTCA procedure.

In still further accordance with the invention, the radiopaque marker can be comprised of at least two semi-cylindrical components, each component associated with the elongated body without obstructing the wall discontinuity.

In accordance with a further aspect of the invention, the radiopaque marker can have a variety of constructions, including but not limited to a cylinder, coil, torus, filament, band, and ink.

In still further accordance with an example, a method for associating the radiopaque marker with the elongated body can be provided that includes the steps of applying a cyanoacrylate adhesive to one or both components, placing the components in contact, and optionally, creating a bead of cyanoacrylate adhesive adjacent the edges of the radiopaque marker in order to create a smooth transition.

In further accordance with an example, a method can be provided for associating the radiopaque marker with the elongated body using a welding, heat staking, heat bonding, or swaging process.

In an alternative embodiment of the present invention, the guidewire channels are defined by the inner wall thickness of the elongated body and the inner surface of a guiding catheter that the guidewire placement device is inserted within. In further accordance with this embodiment, the cross section of the elongated body can have a variety of shapes for example, but not limited to an s-curve, cross, and spiral, the outer surface of said cross section defining the inner wall thickness of the elongated body.

In further accordance with an example, a method can be provided for applying a lubricious material to the outer surface of the elongated body and to the guidewire channel in order to improve guidewire trackability.

In accordance with the present invention, the elongated body may be composed of materials that provide acceptable trackability. Materials that can be provided to meet this need include, but are not limited to, nylon, polyamide, PVC, Pebax, PTFE, FEP, and urethane.

In still further accordance with the present invention, the elongated body can include a loading channel 200 sized to receive a stiffening mandrel. A stiffening mandrel can be inserted within said lumen in order to impart beneficial structural characteristics to enhance trackability of the guidewire placement device.

### BRIEF DESCRIPTION OF THE DRAWINGS

For a further appreciation of the above and other advantages, reference is made to the following detailed description and to the drawings, in which:

Figure 1 is a plan view of an exemplary embodiment of the guidewire placement device in accordance with the present invention;

Figure 1A is a cross sectional view of the exemplary embodiment of the guidewire placement device according to the present invention taken about line A-A of Figure 1;

Figures 2A through 2D are perspective views of alternative embodiments of radiopaque marker designs in accordance with the present invention;

Figure 3 is a partial-perspective view of a distal end portion of the guidewire placement device in accordance with the present invention illustrating an exemplary embodiment of a radiopaque marker disposed thereon;

Figure 4 is a cross sectional view of an alternative embodiment of a guidewire placement device in accordance with the present invention, the alternative embodiment having a loading channel disposed therein;

Figure 5 is a plan view of an alternative embodiment of a guidewire placement device in accordance with the present invention;

Figure 5A is a cross sectional view of the alternative embodiment of the guidewire placement device of Figure 5 taken about line B-B of Figure 5;

Figure 6 is an end view of the alternative embodiment of the guidewire placement device of Figure 5, wherein the alternative embodiment of the guidewire placement device is shown disposed in a guiding catheter, and

Figure 7A through 7B are end views of other alternative embodiments of guidewire placement devices in accordance with the present invention.

### DETAILED DESCRIPTION OF THE PREFERRED EMBODIMENTS

While the present invention will be described with reference to a few specific embodiments, the description is illustrative of the invention and is not to be construed as limiting the invention. Various modifications to the present invention can be made to the preferred embodiments by those skilled in the art without departing from the scope of the invention as defined by the appended claims. It will be noted here that for a better understanding, like components are designated by like reference numerals throughout the various accompanying figures.

In accordance with the present invention there is provided a guidewire placement device to facilitate the placement of guidewires during interventional procedures. The device is particularly useful in assisting the placement of guidewires within branches of a tubular system, such as arteries or vessels, such as during the treatement of bifurcated vessels. The guidewire placement device mitigates guidewire entanglement and knotting that can occur when more than one guidewire is disposed within a lumen.

In accordance with the present invention there is provided a guidewire placement device, wherein the guidewire placement device generally includes an elongated body having a proximal end and a distal end, wherein two lumens are disposed through the elongated body and extend between the proximal and distal ends of the elongated body. Each of the lumens includes a discontinuity formed in a wall of the lumen, thereby causing the lumens to be in fluid communication with an outer surface of the elongated member.

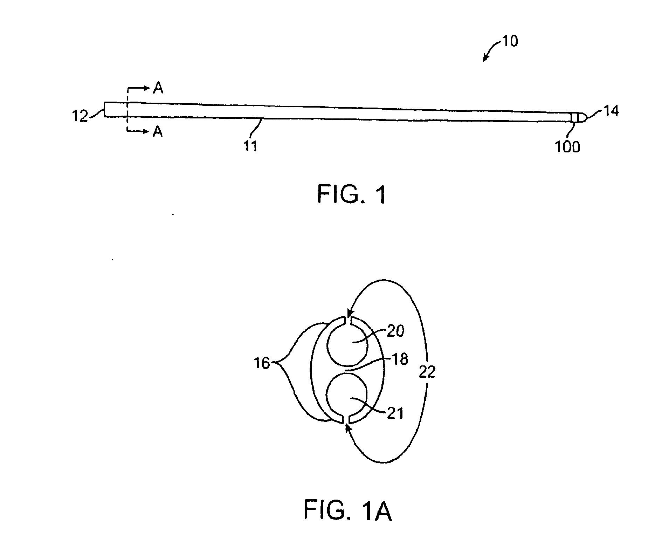

Referring now to Figure 1, there is shown an exemplary embodiment of the guidewire placement device 10 in accordance with the present invention. The guidewire placement device 10 includes an elongated body 11 having a proximal end 12 and a distal end 14. The elongated body 11 is sized to be disposed within a guiding catheter (not shown) and preferably has a generally uniform cross section along the length of the elongated body 11.

It is contemplated that the cross sectional profile of the elongated body 11 can vary along the length of the elongated body 11. For example, the elongated body 11 may taper from the proximal end 12 to the distal end 14. This can vary the stiffness of the elongated body 11, which may affect the trackability of the guidewire placement device 10. In addition, a larger profile of the proximal end 12 can make insertion of guidewires into the guidewire lumens easier as will be described in detail below.

The elongated body 11 may be constructed of a material that provides sufficient kink resistance and trackability. For example, the elongated body 11 may be constructed of Nylon. Other suitable materials for construction of the elongated body 11 include: polyamide, Pebax, silicone, PVC, polyurethane, urethane, PTFE, polyethylene, or any combination thereof. It is further contemplated that a reinforcement material may be added during construction of the elongated body 11, where the reinforcement material may be disposed along the entire length of the elongated body or along a portion of the elongated body as desired. Examples of suitable reinforcement materials include wire mesh, wire braiding, nylon braiding or similar reinforcement techniques known to one of ordinary skill in the art.

In further accordance with the present invention, the elongated body 11 may be constructed as a unitary member from a single material in order to provide uniform material characteristics throughout. Further still, the elongated body 11 may be constructed from multiple materials that are varied along its length to provide a stiffness gradient. The material of the elongated body 11 can be varied to achieve desirable performance characteristics.

As shown in Figure 1, the distal tip 14 of the guidewire placement device 10 is preferably embodied as an atraumatic tip. The tip 14 is preferably formed having round edges at its distal end to form the traumatic tip. Alternatively, filleted edges may be used to ensure that the tip is atraumatic to the vessel wall when the device is being tracked through tortuous anatomy. The material at the distal tip may be formed from a low durometer or plasticized to make it atraumatic. Additionally, as shown in Figure 1, the guidewire placement device 10 further includes a radiopaque marker 100 disposed adjacent to the tip 14. Various embodiments of the marker 100 will be described in greater detail below with regard to additional drawing figures.

Referring now to Figure 1A there is shown a cross-sectional view of the guidewire placement device 10 in accordance with the present invention taken about line A-A of Figure 1. As shown in Figure 1A, the elongated body 11 is generally formed as a solid member including at least two lumens 20 and 21 extending therethrough. The lumens 20,21 define an outer wall thickness 16 and an inner wall thickness 18. In a further aspect of the present embodiment, the inner wall thickness 18 separates the lumens 20 and 21 from each other.

Referring again to Figure 1A, as shown, the guidewire placement device 10 further includes discontinuities 22 formed in the outer wall thickness 16. These discontinuities 22 provide communication between the lumens 20, 21 and the space that is external to the guidewire placement device 10. In the present embodiment, each discontinuity 22 is positioned opposite from the other, and located at the point of minimum outer wall thickness 16. Alternatively, each discontinuity 22 may be positioned symmetrically or asymmetrically relative to the other, and not in a location of minimum outer wall thickness 16.

In further accordance with the present invention, each discontinuity 22 may extend along the entire length of the elongated body or terminate at a location adjacent to a proximal end 12 of the elongated body 11. Each discontinuity 22 follows a path distally along the elongated body 11, terminating at the distal end 14 of the elongated body 11. In doing so, a continuous discontinuity 22 is created, allowing a guidewire to pass from the guidewire channels 20 and 21 through the outer wall thickness 16 over the entire length of the elongated body 11. The discontinuity is preferably formed having a width less than that of a guidewire, which may be disposed within either of the lumens 20, 21.

In an alternative example, the discontinuity 22 may terminate at the proximal end 12 of the elongated body 11 and have a distal terminus that is proximal to the distal end 14 of the elongated body 11.

The discontinuity 22 may be formed in the outer wall thickness 16, by passing a cutting apparatus either externally into the lumens 20, 21 or drawing the cutting apparatus through the guidewire channels 20 and 21 to form the discontinuity.

Alternatively, the discontinuity 22 in the outer wall thickness 16, may be formed by supporting the guidewire channels 20 and 21 with a mandrel, and then passing the outer wall thickness 16 between the mandrel and a laser source, the laser source directing a laser beam through the outer wall thickness 16 and thereby creating the discontinuity 22. In a further aspect of the alternative embodiment a variety of laser sources may be used, for example but not limited to, Nd-YAG, excimer, nanosecond, femtosecond, and CO2 lasers. The laser may also be replaced by a suitable focused energy source, such as a water jet.

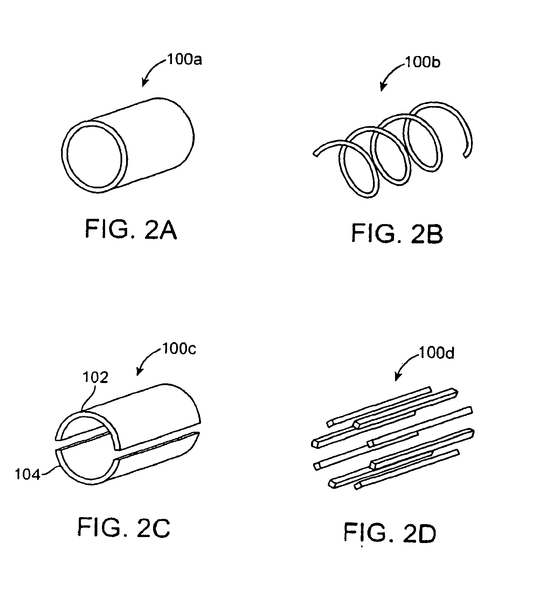

Referring now to Figures 2A through 2D, there are shown in accordance with the present invention various embodiments for the radiopaque marker 100 of the guidewire placement device 10 in accordance with the present invention and as previously described.

Referring now to Figure 2A, there is shown one example of a radiopaque marker in accordance with the present invention. As shown in Figure 2A, marker 100a has a cylindrical configuration, which allows it to be placed in association with the entire circumference of the elongated body 11. It can be appreciated that with this configuration, the discontinuity 22 in the outer wall thickness 16 will be obstructed, and therefore, a guidewire cannot pass from the guidewire channels 20 and 21 to the space that is external to the guidewire placement device 10. However, the marker can be cut axially to allow the guidewire to pass out of the gas channels.

Referring now to Figure 2B, there is shown a second alternative example of a radiopaque marker 100b in accordance with the present invention. As shown in Figure 2B, the radiopaque marker 100b is embodied as a coiled configuration. In this configuration, the marker can be placed in association with the entire circumference of the elongated body 11. As described above with regard to the embodiment shown in Figure 2B, the radiopaque marker 100b will also obstruct the discontinuities 22 in the outer wall thickness 16. However, the marker can be cut axially to allow the guidewire to pass out of the gas channels.

Referring now to Figure 2C, there is shown a third example of a radiopaque marker in accordance with the present invention. As shown in Figure 2C, the radiopaque marker 100c is comprised of a first half 102 and a second half 104, wherein the first and second halves have a radius of curvature substantially equal to that of the elongated member 11. Each of the halves is preferably affixed to the outer surface of the elongated member 11 at a desired location. When assembled, the halves do not encircle the entire circumference of the elongated shaft 11, thereby leaving the discontinuities 22 unobstructed.

Referring now to Figure 2D, there is shown a fourth alternative example of the radiopaque marker in accordance with the present invention. As shown in Figure 2D, the radiopaque marker 100d is formed from at least longitudinal member, and more preferably a plurality of longitudinal members. Each longitudinal member being substantially aligned with a longitudinal axis of the elongated member 11. The longitudinal members can be associated with the elongated member so as not to obstruct the discontinuity 22 in the outer wall thickness 16 and thereby allowing a guidewire to pass from the guidewire channels 20 and 21 through the discontinuity 22.

The radiopaque markers shown in Figures 2A through 2D and described above are preferably affixed to an outer surface of the elongated shaft with a biocompatible adhesive. This adhesive can be a time or light cured cyanoacrylate that is applied to the inner surface of the radiopaque marker 100 and/or the outer surface of the elongated body 11. The marker 100 and elongated body 11 can then be placed in contact, allowing the adhesive to cure, and resulting in a bond between the components. In further accordance with the present embodiment, a bead of adhesive can be placed adjacent the edges of the radiopaque marker 100 and the adhesive can then be cured. The cured adhesive bead creates a smooth transition between the surface of the marker 100 and the elongated body 11, which prevents damage to the vessel when the guidewire placement device 10 is tracked through anatomy.

Alternatively, the radiopaque marker 100 may be affixed to the elongated shaft using a heat welding process. In this process, the radiopaque marker 100 is placed in contact with the elongated body 11 and heat is applied to the radiopaque marker. Due to the lower melting temperature of the material of which the elongated member is fabricated of, the elongated member would melt and flow into and about the radiopaque marker 100. The heat can be removed from the components, resulting in a finished assembly. In further accordance with this embodiment, a heat shrink tube may be placed over the assembly prior to heat application. After the heat is applied, the heat shrink tubing will undergo a reduction in diameter and will come in contact with the radiopaque marker 100 and the surface of the elongated body 11. The material of the elongated body 11 will melt and flow within the heat shrink tubing when the heat is applied. The melted material will flow into and about the radiopaque marker 100, and will result in a smooth transition at the marker edges, due to the conformance provided by the heat shrink tubing. Mandrels may be placed within the lumens and or the discontinuities so that the lumens and the discontinuities remain patent during this process.

In still another example, there is provided a method for affixing the radiopaque marker 100 to the elongated body 11 using a swaging process. In this example, a marker component such as the one illustrated in Figure 2A may be utilized. Since the marker component has a continuously circumferential material configuration, it is possible to deform the material into a smaller diameter by applying a radial force using a swaging process, for example. Therefore, the radiopaque marker 100 may be placed on the elongated body 11 in the desired location. Next, a radial force may be applied to the radiopaque marker, thereby deforming the marker and placing it in interference contact with the elongated member. The interference contact between the components secures the marker to the elongated member, resulting in a final assembly. Optionally, a mandrel may be placed within one or all of the guidewire channels 20 and 21 of the elongated member during the swaging process.

It is further contemplated that the radiopaque marker 100 in accordance with an example may be embodied in the form of a radiopaque ink The radiopaque ink can be applied to the elongated body 11 surface using known printing methods such as pad printing. If desired, a protective layer may be disposed over the radiopaque ink, wherein the protective layer may be comprised of a curable adhesive or heat shrink material. The protective layer may also be a film secured over the radiopaque ink by an adhesive or heat welding prcess.

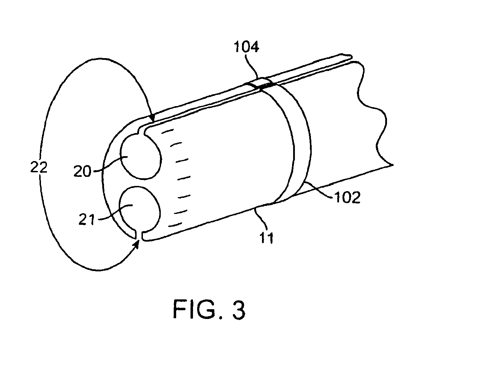

Referring now to Figure 3 there is shown a partial isometric view of the distal end of the guidewire placement device 10 in accordance with the present invention. As shown in Figure 3, a radiopaque marker as shown in Figure 2B is shown disposed adjacent the distal end of the guidewire placement device 10. As described above, this example of the radiopaque marker includes two components 102 and 104, each of which are semi-cylindrical in form. As shown in Figure 3, these marker halves can each be placed in association with the elongated body 11 such that the discontinuities in the outer wall thickness 16 of the elongated body 11 are not obstructed. This permits a guidewire to pass from the guidewire channels 20 and 21 through the discontinuity 22 in the outer wall thickness 16 of the elongated body 11.

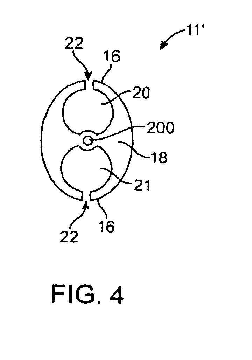

Referring now to Figure 4, there is shown an end view of an alternative embodiment the guidewire placement device in accordance with the present invention. As shown in Figure 4, the elongated body 11' according to the alternative embodiment further includes a loading channel 200. This loading channel 200 can be used to insert a stiffening mandrel (not shown). The mandrel may be constructed from a material that is stiffer than the material used to construct the elongated body 11'. For example, the mandrel may be stainless steel, nitinol, or a glass filled delrin or other biocompatible materials or composites. The stiffer mandrel imparts structural characteristics to the overall assembly that provides better kink resistance and trackability. Although the loading channel 200 in this figure is shown at a location within the inner wall thickness 18' of the elongated body 11', in further embodiments, the loading channel 200 may be placed in a location that is not coaxial with the longitudinal axis defined by the elongated body 11. It is also possible to use a stiffening mandrel that is softer than the elongated body material. The composite structure will still act to provide an over all increase or stiffness.

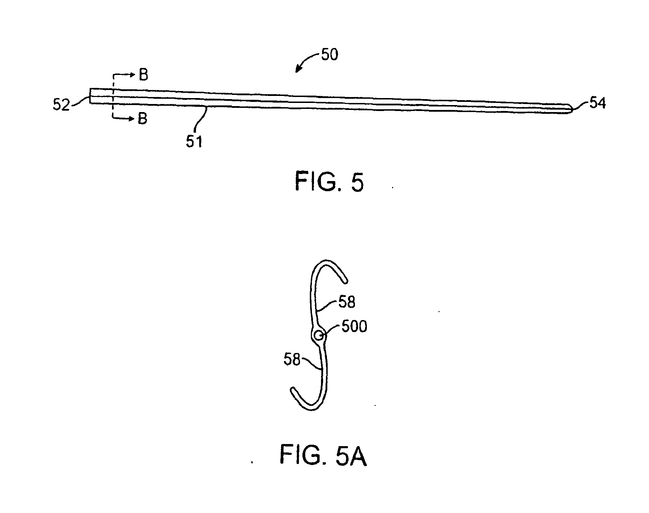

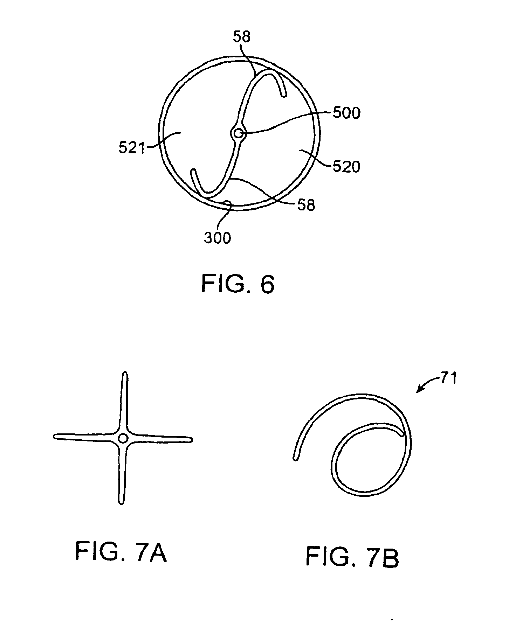

Referring now to Figure 5, there is shown yet another alternative example of a guidewire placement device 50 in accordance with the present invention. According to the example shown in Figure 5, the guidewire placement device 50 comprises an elongated body 51 with a proximal end 52 and a distal end 54. Referring now to Figure 5A there is shown a cross sectional view of the elongated body 51, taken along section line B-B of Figure 5. As illustrated by this view, the elongated body 51 includes a loading channel 500 sized to accept a mandrel formed from a stiffer material, as described in a previous example. In addition, a radiopaque material may be inserted within the loading channel 500 both to provide stiffness, and to function as a marker at various points, such as the catheter tip. In the present example, the elongated body 51 has a substantially "S-Curved" profile. The surface of this profile defines the inner wall thickness 58 of the elongated body 51 in accordance with the present invention. Referring now to Figure 6, there is shown the guidewire placement device of Figure 5 as disposed within a guiding catheter. As shown in Figure 6, guidewire channels 520 and 521 are formed when the elongated body 51 is placed in association with a guiding catheter 300. The guidewire channels 520 and 521 are defined by the inner wall thickness 58 of the elongated body 51 and the inner surface of the guiding catheter 300.

For example, referring now to Figures 7A through 7B, it is possible to have a cross sectional profile that is substantially a "Cross" geometry as shown in Figure 7A. Further still, an elongated body 71 geometry may be used that is substantially a "Spiral" as shown in Figure 7B.

Moreover, the elongated body need not include a loading channel or stiffening mandrel as described above. For example, and in accordance with an alternative example as depicted in Figure 7B, the elongated body can be embodied in the form of a spiral, wherein as shown, the spiral configuration forms a first lumen and a second lumen as shown.

In further accordance with the present invention, the surface of the elongated body may include a coating configured to increase lubricity. In a further aspect of this embodiment, synthetic or natural oil may be applied to the outer surface of the elongated body and/or within the lumens as described herein to reduce friction and/or increase lubricity. In an alternative embodiment, these surfaces may be treated with a hydrophilic coating.

The guidewire placement device in accordance with the present invention may be utilized to place at least one guidewire into a branch vessel of a vessel system. An example of a branch vessel is a bifurcated coronary vessel. The guidewire placement device of the present invention is preferably utilized in conjunction with a guiding catheter. The guiding catheter having been previously placed within the patient's vasculature. The guidewire placement device is then disposed within the guiding catheter and advanced to a desired position. Positioning of the guidewire placement device may be verified by viewing of the radiopaque marker disposed adjacent the distal end of the guidewire placement device under fluoroscopy.

After having placed the guidewire placement device at a desired location, a first guidewire can then be inserted into one of the lumens of the guidewire placement device and advanced into a first coronary bifurcation branch. A second guidewire can then be placed into the second lumen and advanced into a second coronary bifurcation branch. It can be appreciated that since each guidewire is tracked through a separate guidewire channel they are kept separate over the length of the device by the inner wall thickness 18. Therefore, it is not possible for the guidewires to become entangled, twisted or knotted as may otherwise occur.

After having placed the guidewires, the guidewires can be removed from the lumens of the guidewire placement device. The guidewires may be removed utilizing different methods. One such method is to allow each guidewire to pass through the discontinuity 22 formed in the outer wall thickness 16 of the elongated body 11 to the space that is external to the guidewire placement device 10. To initiate removal in this manner, the guidewires may be removed through the discontinuity formed in the proximal end of the guidewire placement device and propagated along the length of the elongated member until each of the guidewires are free of their respective lumens. "Propogation" may be facilitated by passing a secondary device over the guidewires external to the placement device or by passing a surface distally between the guidewires and the outer surface of the guidewire placement device.

An alternative examplary method of removing the guidewires from their respective lumens is to remove the guidewire placement device from the guiding catheter while retaining the position of each of the guidewires. This may be accomplished by applying an axial force to the guidewire loading device in a proximal direction until the distal end 14 of the elongated body 11 is no longer associated with the guidewires. Optionally, the guidewires are secured proximal to the guiding catheter while applying the axial force, thereby maintaining position of the guidewires within the coronary bifurcation vessels.

This method results in two guidewires that are placed within the branches of a coronary bifurcated vessel having minimal twists or entanglements between them. Thus, the guidewires can be used to perform an interventional technique such as, but not limited to, Provisional T, Culottes, and Crush techniques.

While particular embodiments of the present invention have been shown and described, it will be obvious to those skilled in the art that changes and modifications can be made without departing from this invention in its broader aspects. Therefore, the appended claims are to encompass within their scope all such changes and modifications as fall within the scope of this invention.

## Claims

1. A guidewire placement device (10; 50), comprising:
an elongated body (11; 11'; 51; 71) having a proximal end (12; 52), a distal end (14; 54), an inner wall thickness (18; 58), and an outer wall thickness;
a tip formed at the distal end;
a first lumen (20; 520) for receiving a first guidewire, the first lumen defined in part by the inner wall thickness and extending along a length of the elongated body;
a first discontinuity (22) formed in the outer wall thickness, the first discontinuity associated with the first lumen,
**characterised by**
a second lumen (21; 521) for receiving a second guidewire, the second lumen defined in part by the inner wall thickness and extending along a length of the elongated body;
and a second discontinuity (22) formed in the outer wall thickness associated with the second lumen.

2. The device of claim 1, wherein each lumen is further defined by the outer wall thickness (16) of the elongated body.

3. The device of claim 1, wherein each lumen is further defined by an inner surface of a guiding catheter (300), said guiding catheter being circumferentially associated with the device.

4. The device of claim 3, wherein a radiopaque marker (100; 100a; 100b; 100c; 100d; 102, 104) is associated with the elongated body, the radiopaque marker being constructed from a material that is more radiopaque than that used to form the elongated body.

5. The device of claim 1, further comprising a loading channel (200, 500) in the elongated body, said loading channel being configured to receive a stiffening mandrel.

6. The device of claim 5, wherein the stiffening mandrel is constructed from a material selected from the group of stainless steel, nitinol, or glass-filled delrin.

7. The device of claim 1, wherein the elongated body is at least partially constructed from a material selected from the group consisting of nylon, polyamide, Pebax, silicone, PVC, PTFE, FEP, urethane or polyurethane.

8. The device of claim 7, wherein the elongated body is constructed from more than one material and wherein each of the materials provides a degree of stiffness along the length of the elongated body.

9. The device of claim 1, wherein the tip formed at the distal end is atraumatic.

10. The device of claim 9, wherein the atraumatic tip has rounded edges.

11. The device of claim 9, wherein the atraumatic tip has filleted edges.

12. The device of any of claims 1-11, wherein the first and/or second discontinuity begins at the distal end and either extends along the entire length of the elongated body or terminates before the proximal end.

## Patentansprüche

1. Vorrichtung zur Platzierung von Führungsdrähten (10; 50) mit:
einem länglichen Körper (11; 11'; 51; 71), der ein proximales Ende (12; 52), ein distales Ende (14; 54), eine Innenwandstärke (18; 58) und eine Außenwandstärke besitzt;
einer am distalen Ende geformten Spitze;
einem ersten Lumen (20; 520) zur Aufnahme eines ersten Führungsdrahtes, wobei das erste Lumen zum Teil durch die Innenwandstärke bestimmt wird und sich entlang einer Länge des länglichen Körpers erstreckt;
einer ersten Diskontinuität (22) in der Außenwandstärke, wobei die erste Diskontinuität mit dem ersten Lumen verbunden ist,
**gekennzeichnet durch**
ein zweites Lumen (21; 521) zur Aufnahme eines zweiten Führungsdrahtes, wobei das zweite Lumen zum Teil **durch** die Innenwandstärke bestimmt wird und sich entlang einer Länge des länglichen Körpers erstreckt;
und eine zweite Diskontinuität (22) in der Außenwandstärke, die mit dem zweiten Lumen verbunden ist.

2. Vorrichtung nach Anspruch 1, worin jedes Lumen weiterhin durch die Außenwandstärke (16) des länglichen Körpers bestimmt wird.

3. Vorrichtung nach Anspruch 1, worin jedes Lumen weiterhin durch eine Innenfläche eines Führungskatheters (300) bestimmt wird, und der besagte Führungskatheter entlang eines Umfangs mit der Vorrichtung verbunden ist.

4. Vorrichtung nach Anspruch 3, worin ein röntgenstrahlenundurchlässiger Marker (100; 100a; 100b; 100c; 100d; 102; 104) mit dem länglichen Körper verbunden ist, wobei der röntgenstrahlenundurchlässige Marker aus einem Material hergestellt wird, das röntgenstrahlenundurchlässiger als das für das Formen des länglichen Körpers benutzte Material ist.

5. Vorrichtung nach Anspruch 1, die weiterhin einen Ladekanal (200, 500) im länglichen Körper besitzt, wobei der Ladekanal dafür ausgelegt ist, einen Versteifungsmandrin aufzunehmen.

6. Vorrichtung nach Anspruch 5, wobei der Versteifungsmandrin aus einem Material hergestellt wird, das aus der Gruppe bestehend aus rostfreiem Stahl, Nitinol und glasgefülltem Delrin ausgewählt wird.

7. Vorrichtung nach Anspruch 1, wobei der längliche Körper zumindest teilweise aus einem Material hergestellt wird, das aus der Gruppe bestehend aus Nylon, Polyamid, Pebax, Silikon, PVC, PTFE, FEP, Urethan und Polyurethan ausgewählt wird.

8. Vorrichtung nach Anspruch 7, wobei der längliche Körper aus mehr als einem Material hergestellt wird und worin jedes Material ein Steifheitsgrad in Längsrichtung des länglichen Körpers aufweist.

9. Vorrichtung nach Anspruch 1, wobei die am distalen Ende geformte Spitze atraumatisch ist.

10. Vorrichtung nach Anspruch 9, wobei die atraumatische Spitze abgerundete Kanten besitzt.

11. Vorrichtung nach Anspruch 9, wobei die atraumatische Spitze verrundete Kanten besitzt.

12. Vorrichtung nach einem der Ansprüche 1-11, wobei die erste und/ oder zweite Diskontinuität am distalen Ende beginnt und sich entweder über die gesamte Länge des länglichen Körpers erstreckt oder vor dem proximalen Ende aufhört.

## Revendications

1. Dispositif de mise en place de fil-guide (10 ; 50), comprenant :
un corps allongé (11 ; 11' ; 51 ; 71) comportant une extrémité proximale (12 ; 52), une extrémité distale (14 ; 54), une épaisseur de paroi interne (18 ; 58), et une épaisseur de paroi externe ;
une pointe formée au niveau de l'extrémité distale ;
une première lumière (20 ; 520) destinée à recevoir un premier fil-guide, la première lumière définie en partie par l'épaisseur de paroi interne et s'étendant le long d'une longueur du corps allongé ;
une première discontinuité (22) formée dans l'épaisseur de paroi externe, la première discontinuité associée à la première lumière,
**caractérisé par**
une deuxième lumière (21 ; 521) destinée à recevoir un deuxième fil-guide, la deuxième lumière définie en partie par l'épaisseur de paroi interne et s'étendant le long d'une longueur du corps allongé ;
et une deuxième discontinuité (22) formée dans l'épaisseur de paroi externe associée à la deuxième lumière.

2. Dispositif selon la revendication 1, dans lequel chaque lumière est en outre définie par l'épaisseur de paroi externe (16) du corps allongé.

3. Dispositif selon la revendication 1, dans lequel chaque lumière est en outre définie par une surface interne d'un cathéter de guidage (300), ledit cathéter de guidage étant associé sur une circonférence au dispositif.

4. Dispositif selon la revendication 3, dans lequel un marqueur radio-opaque (100 ; 100a ; 100b ; 1 00c ; 100d ; 102, 104) est associé au corps allongé, le marqueur radio-opaque étant construit à partir d'un matériau qui est plus radio-opaque que celui utilisé pour former le corps allongé.

5. Dispositif selon la revendication 1, comprenant en outre un canal de chargement (200, 500) dans le corps allongé, ledit canal de chargement étant conçu pour recevoir un mandrin de raidissement.

6. Dispositif selon la revendication 5, dans lequel le mandrin de raidissement est construit à partir d'un matériau choisi parmi le groupe de l'acier inoxydable, du nitinol, ou de Delrin rempli de verre.

7. Dispositif selon la revendication 1, dans lequel le corps allongé est au moins partiellement construit à partir d'un matériau choisi dans le groupe constitué de nylon, polyamide, Pebax, silicone, PVC, PTFE, FEP, uréthane ou polyuréthane.

8. Dispositif selon la revendication 7, dans lequel le corps allongé est construit à partir de plus d'un matériau et dans lequel chacun des matériaux fournit un degré de rigidité le long de la longueur du corps allongé.

9. Dispositif selon la revendication 1, dans lequel la pointe formée à l'extrémité distale est atraumatique.

10. Dispositif selon la revendication 9, dans lequel la pointe atraumatique a des bords arrondis.

11. Dispositif selon la revendication 9, dans lequel la pointe atraumatique a des bords filetés.

12. Dispositif selon l'une quelconque des revendications 1 à 11, dans lequel les première et/ou deuxième discontinuités commencent à l'extrémité distale et soit s'étendent sur toute la longueur du corps allongé soit se terminent avant l'extrémité proximale.
